(19) **Europäisches Patentamt European Patent Office Office européen des brevets**

(11) **EP 1 538 987 B1**

(12) **FASCICULE DE BREVET EUROPEEN**

(45) Date de publication et mention
de la délivrance du brevet:
**21.12.2011 Bulletin 2011/51**

(51) Int Cl.:
***A61B 8/08*** (2006.01)

(21) Numéro de dépôt: **03769557.4**

(22) Date de dépôt: **02.09.2003**

(86) Numéro de dépôt international:
**PCT/FR2003/002630**

(87) Numéro de publication internationale:
**WO 2004/021888 (18.03.2004 Gazette 2004/12)**

(54) **DISPOSITIF ET PROCEDE POUR LA MESURE DE L ELASTICITE D UN ORGANE HUMAIN OU ANIMAL ET L ETABLISSEMENT D UNE REPRESENTATION A DEUX OU TROIS DIMENSIONS DE CETTE ELASTICITE**

VORRICHTUNG UND VERFAHREN ZUR MESSUNG DER ELASTIZITÄT EINES MENSCHLICHEN ODER TIERISCHEN ORGANS UND ZUR ERZEUGUNG EINER ZWEI- ODER DREIDIMENSIONALEN DARSTELLUNG DIESER ELASTIZITÄT

DEVICE AND METHOD FOR MEASURING ELASTICITY OF A HUMAN OR ANIMAL ORGAN AND FOR TWO- OR THREE-DIMENSIONAL REPRESENTATION THEREOF

(84) Etats contractants désignés:
**AT BE BG CH CY CZ DE DK EE ES FI FR GB GR HU IE IT LI LU MC NL PT RO SE SI SK TR**

(30) Priorité: **06.09.2002 FR 0211074**

(43) Date de publication de la demande:
**15.06.2005 Bulletin 2005/24**

(73) Titulaire: **Echosens
75013 Paris (FR)**

(72) Inventeurs:
• **SANDRIN, Laurent**
**94240 L'Hay-les-Roses (FR)**
• **HASQUENOPH, Jean-Michel**
**F-77860 Couilly-Pont-aux-Dames (FR)**
• **YON, Sylvain**
**F-75005 Paris (FR)**

(74) Mandataire: **Lebkiri, Alexandre
Cabinet Camus Lebkiri
10 rue de la Pépinière
75008 Paris (FR)**

(56) Documents cités:
WO-A-00/55616          WO-A-01/76484
WO-A-02/35256          FR-A- 2 733 142
US-A- 5 099 848        US-A- 5 474 070
US-A1- 2002 010 398    US-B1- 6 176 827
US-B1- 6 277 074

• BERCOFF J ET AL: "Ultrafast compound imaging for 2d displacement vector measurements: application to transient elastography and color flow mapping" 2001 IEEE ULTRASONICS SYMPOSIUM PROCEEDINGS. ATLANTA, GA, OCT. 7 - 10, 2001, IEEE ULTRASONICS SYMPOSIUM PROCEEDINGS, NEW YORK, NY: IEEE, US, vol. 2 OF 2, 7 octobre 2001 (2001-10-07), pages 1619-1622, XP010584823 ISBN: 0-7803-7177-1

**Description**

La présente invention concerne un dispositif et un procédé pour la mesure de l'élasticité d'un organe humain ou animal, ou plus généralement tous milieux viscoélastiques présentant un signal ultrasonore après illumination ultrasonore et l'établissement consécutif d'une représentation à deux ou trois dimensions de l'élasticité. Elle s'applique en particulier, mais non exclusivement, à la mesure de l'élasticité d'un sein humain, l'intérêt de cette technique est que le caractère pathologique des tissus est souvent relié à leur élasticité.

On connaît dans l'art antérieur la demande de brevet français FR 2733142 qui divulgue un dispositif de mesure d'élasticité réalisant une mesure à 2 dimensions mais également apte à réaliser une mesure à trois dimensions. Néanmoins, ce dispositif ne dispose pas d'un moyen de balayage apte à effectuer le balayage de la ou les barrettes suivant deux directions perpendiculaires.

On connaît par ailleurs les brevets américains US 6176827, US 5099848, US 2002/010398, US 6277074, US 5474070 qui divulguent tous des solutions pour faire uniquement une mesure à deux dimensions, avec parfois une barrette fixe (cf. US 6176827)

À l'heure actuelle, il n'existe pas sur le marché de dispositifs ultrasonores de mesure de l'élasticité permettant de visualiser ladite mesure en deux ou trois dimensions.

Par ailleurs, concernant la mesure d'élasticité à deux dimensions, on connaît l'article "Shear Modulus Imaging with 2D Transient Elastography" par Sandrin, L., Tanter, M., Catheline, S., and Fink, M., Ultrason. Ferroelectr. Freq. Control., vol. 49 (4), pp. 426-435 (2002) décrivant une technique de mesure de l'élasticité et la représentation à deux dimensions de cette mesure. La résolution du problème inverse, c'est-à-dire consistant à remonter aux paramètres qui décrivent le milieu viscoélastique que l'on cherche à mesurer, est ici imparfaite car le déplacement n'est pas connu dans les trois directions de l'espace. En effet, selon les algorithmes de calcul liés aux mesures effectuées par le dispositif présenté dans cet article, les opérateurs seront obligés de formuler des hypothèses pour résoudre les calculs d'élasticité, mais la pratique a démontré que ces hypothèses sont rarement justifiées.

On connaît également le brevet américain US 2002/010398 qui divulgue une méthode et un système d'imagerie ultrasore pour déterminer la vitesse de propagation d'ondes de cisaillement générées dans un tissu.

On connaît également la demande internationale de brevet N°WO 0070362 qui décrit un système utilisant l'élastographie par résonance magnétique (ERM), dans laquelle une zone viscoélastique (telle que la poitrine humaine) est excitée par des ondes mécaniques. L'objet de cette invention se base sur l'hypothèse que les résultats des mesures par ERM sont les solutions indépendantes du temps des équations différentielles partielles décrivant avec exactitude le comportement d'ondes mécaniques dans un matériau viscoélastique (y compris pour des ondes longitudinales et dans un environnement réfléchissant). Pour cela, le module de Young contenu dans ces équations peut être calculé. De plus, il est proposé ici d'utiliser (de façon prédominante) des ondes longitudinales, celles-ci étant capables de pénétrer dans la poitrine humaine, ce qui n'est pas le cas des ondes transversales.

Dans le dispositif de cette demande de brevet, l'obtention de la carte d'élasticité nécessite beaucoup de temps. Par ailleurs, le coût de mise en oeuvre de ce dispositif est très élevé.

En outre, il est clair que mesurer l'élasticité sur des dimensions plus importantes constitue un avantage considérable, cette amélioration significative de l'état de l'art étant obtenue grâce au dispositif selon l'invention.

L'art antérieur est aussi constitué par la demande internationale de brevet WO-A-00/55616 qui décrit un procédé d'imagerie pour observer la propagation d'une onde impulsionnelle de cisaillement basse fréquence simultanément en une multitude de points d'un milieu viscoélastique diffusant. À cet effet, on émet, dans ce dispositif, à cadence ultrarapide des ondes ultrasonores de compression qui permettent d'obtenir une succession d'images du milieu, puis on traite en temps différé les images ainsi obtenues par intercorrélation, pour déterminer en chaque point de chaque image les mouvements du milieu lors de la propagation de l'onde de cisaillement.

Cette invention ne donne pas satisfaction car elle nécessite d'envisager deux hypothèses :

- la dérivée seconde du déplacement est considérée nulle dans la direction orthogonale au plan,
- le milieu est supposé parfaitement incompressible.

L'invention a donc plus particulièrement pour but de remédier aux inconvénients des systèmes de l'art antérieur.

L'invention est un dispositif tel que défini dans la revendication 1.

Grâce à ces particularités, l'invention permet donc de proposer un dispositif qui permet d'obtenir une cartographie de l'élasticité du milieu à mesurer en deux ou trois dimensions, grâce à un système relativement simple et peu coûteux relativement aux solutions existantes.

La barrette ultrasonore comprend une pluralité de transducteurs destinés à l'acquisition des signaux ultrasonores.

Selon une possibilité offerte par l'invention, le moyen de d'excitation consistera en une vibration mécanique qui pourra être transversale, longitudinale ou plus généralement un mélange des deux.

Avantageusement, le moyen d'excitation pourra consister en un ou plusieurs transducteurs d'hyperthermie car l'élévation de la température engendre des déplacements sur les images ultrasonores, soit avec le(s) transducteur(s) utilisé(s) pour l'acquisition des signaux ultrasonores soit un ou plusieurs transducteurs disposés autour du milieu viscoélastique.

Selon une autre possibilité offerte par l'invention, le moyen d'excitation consistera en une palpation à distance en utilisant la pression de radiation, soit avec le(s) transducteur(s) utilisé(s) pour l'acquisition des signaux ultrasonores soit un ou plusieurs transducteurs disposés autour du milieu viscoélastique.

Avantageusement, le dispositif selon l'invention sera commandé par au moins un moyen de commande, par exemple un ordinateur, un micro-ordinateur ou une unité centrale.

Avantageusement, la barrette ultrasonore sera une barrette permettant de focaliser en une pluralité de différents points d'élévation ; dans ce cas, le balayage étant réalisée par focalisation ultrasonore.

Il convient ici de rappeler que la barrette est apte non seulement à focaliser suivant un plan mais également en élévation par rapport à ce plan, dans l'exemple selon le plan horizontal parallèle au précédent et légèrement décalé.

On rappelle également, dans un souci de faciliter la compréhension de l'invention, qu'une barrette échographique à 0 D émet suivant une dimension linéaire x, qu'une barrette 1 D émet suivant un plan à deux dimensions x, y et enfin qu'une barrette 2 D, constituée habituellement par une multitude de transducteurs ultrasonores de forme carrée répartis suivant une matrice à 2 D, permet d'émettre des ultrasons dans un volume suivant les trois dimensions x, y et z.

Selon un mode d'exécution de l'invention, l'espace existant entre la barrette ultrasonore et le susdit milieu viscoélastique sera constitué au moins en partie par de l'eau ou tout autre élément apte à assurer le libre passage des ondes ultrasonores.

Avantageusement, l'ensemble constitué des transducteurs ultrasonores et de leur électronique embarquée sera relié au moyen de commande et de traitement par une liaison numérique à très haute vitesse, par exemple de type LVDS.

Selon un mode d'exécution de l'invention, le dispositif selon l'invention comprendra deux barrettes ultrasonores.

Selon un mode de réalisation du dispositif de l'invention, les deux barrettes seront immergées dans un récipient hermétique rempli d'un liquide, par exemple de l'eau.

Avantageusement, le récipient hermétique sera relié à un moyen de rotation apte à faire tourner ledit récipient.

Selon une possibilité offerte par l'invention, le récipient hermétique pourra comprendre une pluralité d'orifices dans lesquelles sont introduits respectivement un vibreur mécanique et/ou un transducteur ultrasonore.

Avantageusement, les orifices sur ou dans le boîtier hermétique seront situés à 90° (degrés) les uns des autres ou l'un de l'autre.

Selon un autre mode d'exécution de l'invention, le dispositif selon l'invention comprendra trois barrettes aptes respectivement à mesurer les vitesses tissulaires suivant les directions y, x et z.

L'invention concerne également un procédé tel que défini dans la revendication 20.

Avantageusement, l'étape de déplacements de la barrette sera répétée autant de fois nécessaire à l'acquisition de l'ensemble des données ultrasonores avant de passer à l'étape de calcul des images ultrasonores.

Il doit être noté que l'étape d'acquisition des données ultrasonores permet également d'acquérir les données nécessaires à l'obtention d'une image ultrasonore classique, c'est-à-dire utilisant un "beamforming" classique. En effet, la ou les images ainsi obtenues constituent une information pertinente à 2D ou à 3D sur la morphologie de l'organe étudié, cette information est tout à fait complémentaire du paramètre d'élasticité.

Avantageusement, au cours de l'étape de calcul des vitesses tissulaires, les dérivées seconde de la composante longitudinale de cette vitesse suivant les trois directions orthogonales de l'espace peuvent être mesurées.

De la même manière, au cours de l'étape de calcul des vitesses tissulaires, les dérivées spatiales des trois composantes, suivant les trois directions de l'espace, de ladite vitesse peuvent être mesurées.

Des modes d'exécution de l'invention seront décrits ci-après, à titre d'exemple non limitatif, avec référence aux dessins annexés dans lesquels :

- la figure 1 illustre le déplacement d'une barrette échographique du dispositif selon l'invention muni d'un moyen de balayage mécanique simple ;

- la figure 2 illustre le déplacement d'une barrette échographique du dispositif selon l'invention muni d'un moyen de balayage mécanique double ;

- la figure 3 illustre le fonctionnement d'une barrette 1,5 D du dispositif selon l'invention muni d'un moyen de balayage par focalisation ultrasonore en élévation ;

- la figure 4 illustre le dispositif selon l'invention muni d'une barrette 1,5 ou 1,75 D, apte à focaliser en élévation ;

EP 1 538 987 B1

- la figure 5 illustre le dispositif selon l'invention muni d'un transducteur étoilé dans lequel les transducteurs sont répartis spatialement ;

- La figure 6 illustre le dispositif selon l'invention en train de mesurer l'élasticité du sein d'une patiente ;

- La figure 7 illustre schématiquement un mode de réalisation du dispositif selon l'invention.

Les figures annexées ne représentent pas le dispositif complet. Ce dispositif comprend les éléments usuels pour réaliser des mesures d'élasticité d'un organe humain ou animal, c'est-à-dire notamment au moins une barrette, ou sonde, ultrasonore comportant une pluralité de transducteurs, un équipement électronique apte à assurer l'acquisition des signaux ultrasonores, un moyen de commande et de traitement des données tels qu'un ordinateur ou analogue et un moyen d'excitation apte à engendrer des déplacements basse fréquence.

L'invention se rapporte à l'utilisation d'un moyen de balayage mécanique qui permet d'assurer le balayage de la susdite barrette ultrasonore. Ceci permet, grâce au procédé de l'invention, de mesurer des paramètres qui ne sont pas accessibles avec les dispositifs de l'art antérieur, notamment celui décrit dans le brevet N°FR 9903157. Les paramètres ainsi obtenus sont la dérivée seconde du déplacement suivant l'élévation, c'est-à-dire la direction perpendiculaire au plan de l'image, et les deux composantes manquantes du vecteur déplacement.

Dans la suite, on a choisi d'illustrer l'invention en prenant comme organe humain ou animal, un sein mais tout autre organe, idéalement statique, pourra faire l'objet d'une mesure d'élasticité grâce au dispositif et au procédé selon la présente invention à condition, bien entendu, de présenter un signal ultrasonore après qu'il ait été illuminé à l'aide de signaux ultrasonores. Néanmoins, dans le cas où les mouvements internes du corps ne peuvent constituer une sollicitation basse fréquence utilisable pour le procédé, il est préférable que cet organe soit immobile pour ne pas perturber la mesure.

Le procédé selon l'invention réalise les étapes ci-après selon l'ordre chronologique suivant :

1. génération d'une sollicitation, ou signal, basse fréquence,
2. acquisition des données ultrasonores,
3. déplacement de la barrette grâce au moyen de balayage,
4. calcul des images ultrasonores,
5. calcul des vitesses tissulaires, également dénommé déplacement entre images successives,
6. éventuellement calcul des vitesses de déformation tissulaires,
7. enfin, inversion des données, ce qui permet de récupérer les paramètres du milieu mesuré.

Il doit être noté que les étapes de calcul, soit les étapes 4 à 6, peuvent commencer dès lors que la barrette ultrasonore balaye le milieu viscoélastique, c'est-à-dire que ces étapes ont lieu idéalement pendant le déplacement de ladite barrette.

Au cours de l'étape de génération de la sollicitation, ou signal, basse fréquence, un signal basse fréquence est transmis au moyen d'excitation de préférence juste après le début des acquisitions ultrasonores. Ce signal a une fréquence, f, comprise entre 5 Hz et 1000 Hz. La vibration basse fréquence entraîne la propagation dans les tissus du milieu viscoélastique d'ondes élastiques basse fréquence dont la propagation dépend de l'élasticité du milieu.

Les différents moyens utilisables afin d'engendrer des déplacements basse fréquence peuvent consister en une vibration mécanique, réalisée par un vibreur pouvant être notamment une ou plusieurs plaques vibrantes 20, piston (s) et/ou barre(s). De la même manière, le moyen d'excitation apte à générer une onde de cisaillement pourra consister en une palpation à distance en utilisant la pression de radiation soit avec le(s) transducteur(s) utilisé(s) pour l'acquisition des signaux ultrasonores, soit un ou plusieurs transducteurs disposés autour de l'objet à imager.

Au cours de l'étape d'acquisition des données ultrasonores, N acquisitions ultrasonores sont réalisées à une cadence 1/T typiquement comprise entre 100 Hz et 100 000 Hz. L'acquisition des données ultrasonores se fait en émettant avec les transducteurs ultrasonores une impulsion ultrasonore brève qui est réfléchie par les particules contenues dans le milieu. Le signal ultrasonore, dénommé « speckle », est enregistré par les mêmes transducteurs ultrasonores sur une durée pouvant varier entre 1 μs et 10 ms. Cette opération est répétée un nombre N de fois à la cadence 1/T.

Ensuite survient l'étape de déplacement de la barrette échographique ou ultrasonore. À ce niveau, le balayage consistant à déplacer ladite barrette sera réalisé de trois manières différentes en fonction du nombre et du type de barrette ultrasonore utilisée.

Ainsi, le dispositif selon l'invention pourra notamment être équipé avec :

- une seule barrette ultrasonore unidirectionnelle 1,
- deux barrettes ultrasonores 5, 6 ou une barrette déplacée alors suivant deux axes,

4

- une barrette ultrasonore de type 1,5 D 9.

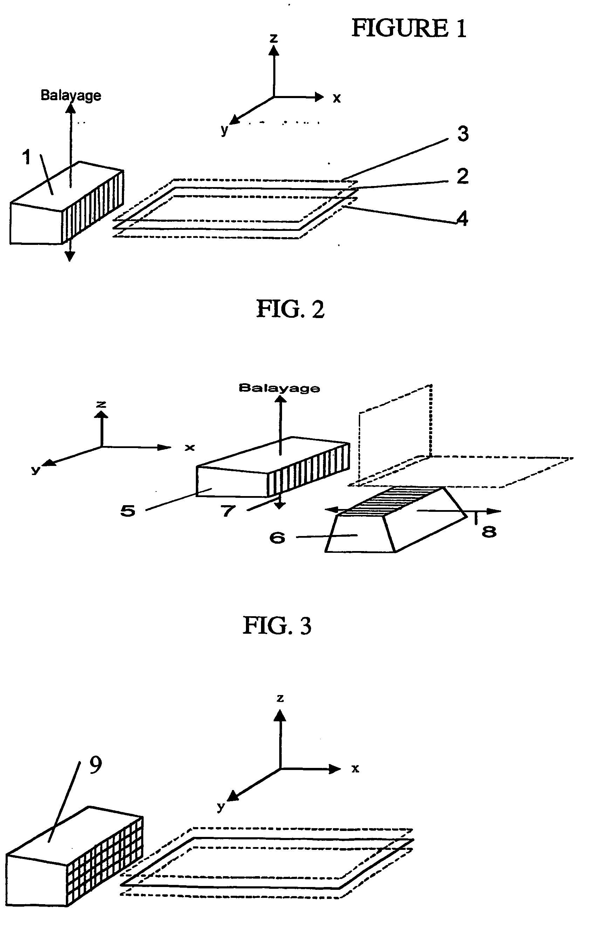

Dans le cas d'une seule barrette ultrasonore unidirectionnelle 1, représenté sur la figure 1, la barrette échographique 1 est déplacée d'une distance comprise entre 10 μm et 10 mm. On réalise au minimum un balayage suivant une direction. Par exemple on balaye suivant la direction z, constitué par le plan 2, en se déplaçant de Δz, constitués sur la figure par les deux plans 3 et 4.

Dans le cas de deux barrettes ultrasonores 5 et 6, représenté sur la figure 2, ou équivalent à une barrette déplacée suivant deux axes 7 et 8, deux barrettes 5 et 6 sont utilisées (ou une seule successivement). Ce balayage permet d'accéder à toutes les composantes du vecteur vitesse tissulaire.

Dans le cas d'une barrette ultrasonore de type 1,5 D 9, représentée sur la figure 3, le balayage mécanique est évité, le résultat étant le même avec un transducteur étoilé ; ces deux éléments permettant de focaliser en trois différents points d'élévation. Dans le cas d'une barrette 1.5D 9, le déplacement suivant z est obtenu en modifiant les lois de focalisation de manière à changer l'élévation du plan de l'image.

Au cours de l'étape de calcul des images ultrasonores, Les images ultrasonores sont construites en utilisant un algorithme de sommation-retard comme celui décrit dans le brevet WO-A-00/55616 précédemment mentionné ou d'autres types de « beamforming » rapide comme par exemple la technique dans l'espace des fréquences spatiales (voir article de Lu, J., « 2D and 3D High Frame Rate Imaging with Limited Diffraction Beams », IEEE Trans. Ultrason. Ferroelectr. Freq. Contr., vol. 44, N°4, 1997.).

Au cours de l'étape de calcul des vitesses tissulaires, également dénommé déplacement entre images successives, les vitesses tissulaires ou déplacements entre deux tirs ultrasonores successifs, mais non nécessairement consécutifs, sont mesurées soit par intercorrélation, décrite dans le brevet WO-A-00/55616, par Doppler, soit par autocorrélation, décrite notamment dans l'article de Kasai C., Namekawa K., Koyano A. et Omoto R. "Real-time two-dimensional blood flow imaging using an autocorrelation technique", IEEE Trans. Sonics Ultrason., vol. 35, pp. 458-464 (1985), et plus généralement par tout autre technique de mesure des déplacements.

En utilisant un balayage mécanique simple représenté sur la figure 1, on accède au moins à la composante suivant x de la vitesse tissulaire $V_x$ en chaque point du milieu situé dans la zone imagée. En utilisant un algorithme du type de celui décrit dans les articles de Konofagou, E. E., Ophir, J., "A new elastographic method for estimation and imaging of lateral displacements, lateral strains, corrected axial strains and Poisson's ratios in tissues", Ultrasound in Med. & Biol. 24, No 8, pp. 1183-1199 (1998) et Tanter, M., Bercoff, J., Sandrin, L., Fink, M., « Ultrafast compound imaging for 2D motion vector estimation : application to transient elastography », Ultrason. Ferroelectr. Freq. Control., on peut accéder également à la composante latérale de la vitesse tissulaire $V_y$. En utilisant un double balayage mécanique tel que représenté sur la figure 2, on accède aux trois composantes de la vitesse tissulaire : la barrette 6 permet de mesurer $V_x$ et $V_y$ et la barrette 5 permet de mesurer $V_y$ et $V_z$. La précision sur l'estimation de $V_y$ est augmentée en calculant la demi-somme des estimations avec les deux barrettes 5 et 6.

Au cours de l'étape optionnelle de calcul des vitesses de déformation tissulaires, la vitesse de déformation tissulaire est obtenue en dérivant $V_z$, également noté v(z,t) suivant la direction de la composante considérée, ici par rapport à la profondeur :

$$\varepsilon_i(z,t) = \frac{\partial v_i(z,t)}{\partial i}$$

avec i = x, y ou z

L'étape d'inversion des données consiste à remonter ou récupérer les paramètres qui décrivent le milieu viscoélastique. Si on considère le milieu linéaire et isotrope, ces paramètres sont au nombre de deux. On peut choisir le module de cisaillement μ et le module de compression λ. En pratique dans les tissus mous λ est de l'ordre du Gpa et varie très peu. μ est de l'ordre du Kpa. L'élasticité ou module d'Young est égale en première approximation à 3μ. Ainsi, il convient de déterminer le module de cisaillement μ qui constitue le paramètre le plus significatif du milieu viscoélastique mesuré.

Dans le cas d'un balayage mécanique simple, c'est-à-dire comportant une barrette unidirectionnelle 1, toutes les composantes du vecteur vitesse tissulaire ne sont pas connues. Les données peuvent être inversées en utilisant l'équation suivante :

$$\rho \frac{\partial^2 v_i}{\partial t^2} = \mu(x,y,z)\left[\frac{\partial^2 v_i}{\partial x^2} + \frac{\partial^2 v_i}{\partial y^2} + \frac{\partial^2 v_i}{\partial z^2}\right]$$

où i= x, y ou z

Pour poser l'équation ci-dessus, il a été nécessaire de faire l'hypothèse que les ondes élastiques parcourant le milieu sont purement des ondes de cisaillement. En pratique, cette hypothèse est fausse car les tissus ne sont pas parfaitement incompressibles, ce qui a pour conséquence que toute onde de cisaillement s'accompagne nécessairement d'une onde de compression.

Le paramètre recherché, $\mu(x,y,z)$, est obtenu en discrêtisant cette équation. En élastographie, on dispose en général d'une des trois coordonnées $v_x$, $v_y$ ou $v_z$. Supposons que ce soit $v_x$. Pour discrétiser cette équation il faut pouvoir calculer les dérivées secondes dans les trois directions et en temps :

$$\begin{cases} \dfrac{\partial^2 v}{\partial^2 x^2} \approx \dfrac{V(j,k+1,l,m)+V(j,k-1,l,m)-2V(j,k,l,m)}{\Delta x^2} \\[2mm] \dfrac{\partial^2 v}{\partial^2 y^2} \approx \dfrac{V(j,k,l+1,m)+V(j,k,l-1,m)-2V(j,k,l,m)}{\Delta y^2} \\[2mm] \dfrac{\partial^2 v}{\partial^2 z^2} \approx \dfrac{V(j,k,l,m+1)+V(j,k,l,m-1)-2V(j,k,l,m)}{\Delta z^2} \\[2mm] \dfrac{\partial^2 v}{\partial^2 t^2} \approx \dfrac{V(j+1,k,l,m)+V(j-1,k-1,l,m)-2V(j,k,l,m)}{T^2} \end{cases}$$

où $V(j,k,l,m) = v(jT, k.\Delta x, y=1.\Delta y, m.\Delta z)$.

Il faut donc non seulement connaître le déplacement $v_x$ dans le plan de l'image, mais également le connaître autour du plan de l'image pour pouvoir estimer la dérivée seconde perpendiculaire au plan de l'image : $\partial v^2/\partial z^2$. Dans le brevet français N°FR 9903157 et les publications en élastographie impulsionnelle, la dérivée seconde perpendiculaire au plan de l'image est éliminée de l'équation car elle ne peut être mesurée expérimentalement. En effet, v n'est mesuré que dans le plan (x,y), seul v (x, y) est connu. $\partial v^2/\partial z^2$ ne peut être déterminée. Une hypothèse connue consiste à poser :

$$\frac{\partial^2 v}{\partial z^2} = 0$$

L'équation se simplifie en

$$\rho \frac{\partial^2 v_i}{\partial t^2} = \mu \left[ \frac{\partial^2 v_i}{\partial x^2} + \frac{\partial^2 v_i}{\partial y^2} \right]$$

où i = x,y ou z.

Elle peut être résolue sans connaître les déplacements dans les plans situés de part et d'autre du plan de l'image en $z+\Delta z$ et $z-\Delta z$.

L'hypothèse de nullité de la dérivée seconde perpendiculaire au plan de l'image est particulièrement contraignante et ne permet pas de résoudre le problème inverse dans de bonnes conditions puisqu'il est hautement improbable que $\partial v^2/\partial z^2$ soit nulle. Grâce au dispositif selon l'invention, la dérivée manquante peut être obtenue.

Il est ainsi envisagé deux solutions pour mesurer v(x, y, z) et calculer $\partial v^2/\partial z^2$ :

- soit on utilise une barrette 1,5 D 9 ou un transducteur étoilé permettant de focaliser en trois différents points d'élévation,
- soit on reproduit trois fois l'acquisition en déplaçant successivement la barrette en $z-\Delta z$, z et $z+\Delta z$ avec $\Delta z$ choisi judicieusement de manière à être voisin des résolutions obtenues en x et y ($\Delta z \cong \Delta x$ et $\Delta y$).

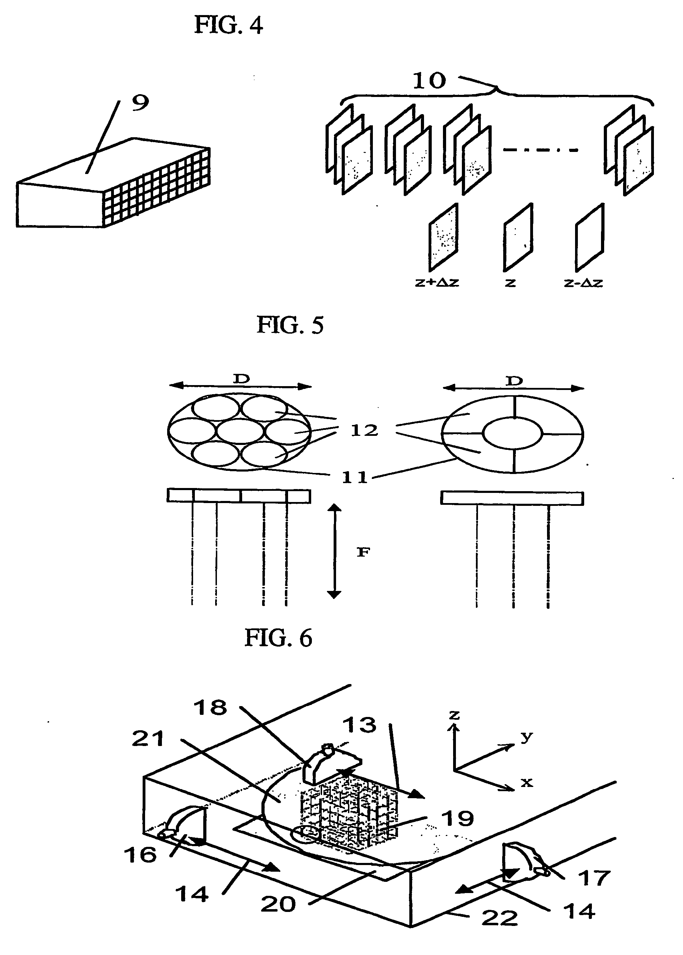

Si on utilise une barrette 1,5 D 9 ou 1,75 D, représenté sur la figure 4, on peut réaliser des images 10 dans trois plans de l'image et calculer les déplacements dans ces trois plans situés par exemple en $z-\Delta z$, z et $z+\Delta z$. la cadence maximale est néanmoins ici réduite d'un facteur 3. On peut également utiliser un transducteur étoilé 11 dans lequel les transducteurs 12 sont répartis spatialement, comme représenté sur la figure 5.

Dans la deuxième solution, consistant à reproduire trois fois l'acquisition en déplaçant successivement la barrette en z-Δz, z et z+Δz, il est à noter qu'il faut veiller à ce que le milieu viscoélasticité à mesurer n'aie pas bougé entre deux acquisitions et à ce que la sollicitation basse fréquence appliquée soit synchronisée pour chaque position en élévation.

Dans le cas d'un balayage mécanique double, c'est-à-dire comportant soit deux barrettes 5, 6 soit une barrette se déplaçant suivant deux axes, toutes les composantes du vecteur vitesse tissulaire sont connues. Un cas plus général (milieu compressible) consiste à utiliser l'équation de Navier qui s'écrit :

$$\rho \frac{\partial^2 \vec{v}}{\partial t^2} = (\lambda + \mu) \vec{\nabla}(\vec{\nabla}.\vec{v}) + \mu \vec{\nabla}^2 \vec{v}$$

On peut affiner ce résultat grâce à l'équation suivante :

$$\rho \frac{\partial^2 v_i}{\partial t^2} = \frac{\partial}{\partial x_i}\left[ \lambda \frac{\partial v_j}{\partial x_j} \right] + \frac{\partial}{\partial x_j}\left[ \mu \left( \frac{\partial v_i}{\partial x_j} + \frac{\partial v_j}{\partial x_i} \right) \right]$$

où $v_1 = v_x$, $v_2 = v_y$, $v_3 = v_z$, $x_1 = x$, $x_2 = y$ et $x_3 = z$.

On a alors un système de trois équations et deux inconnues : $\lambda(x, y, z)$ et $\mu(x, y, z)$ car la densité $\rho$ varie très peu dans les tissus.

Avec l'équation ci-dessus, on comprend pourquoi négliger les vitesses tissulaires liées aux ondes de compression est source d'erreur. Certes les vitesses tissulaires liées aux ondes de compression sont faibles comparées à celles engendrées par l'onde de cisaillement, cependant leur contribution ne peut être négligée car le coefficient $\lambda$ en facteur est grand devant le terme de compression. La discrétisation de cette équation peut être réalisée si les trois composantes du vecteur vitesse tissulaire sont connues. En effet cette équation fait intervenir des couplages entre les évolutions des vitesses tissulaires dans toutes les directions.

L'invention propose d'utiliser un montage ou dispositif tel que représenté sur la figure 6. Ce dispositif permet de mesurer les trois composantes du vecteur vitesse tissulaire dans l'organe étudié en balayant successivement le milieu suivant trois axes différents 13, 14 et 15. La barrette 16 permet de mesurer les vitesses tissulaires suivant la direction y notée $u_y$, la barrette 17 pour mesurer $u_x$ et la barrette 18 pour mesurer $u_z$. L'utilisation d'un algorithme de mesure des déplacements transverses peut permettre de ramener le nombre de zones de balayage de trois à deux en éliminant par exemple la barrette 18. Le déplacement $u_z$ serait alors déterminé à la fois avec la barrette 16 et la barrette 17 ce qui permettrait d'effectuer une moyenne $u_z = (u_{zB1} + u_{zB2}) / 2$.

Un système de synchronisation permet de déplacer le transducteur 12 entre deux acquisitions, une acquisition comprenant la génération des ondes élastiques de cisaillement et l'acquisition des signaux ultrasonores. Le déplacement du système peut être réalisé par exemple avec un moteur pas à pas ou un actionneur électrodynamique.

Cette séquence d'acquisition doit être reproduite autant de fois qu'il y a de plan dans l'image. En utilisant trois barrettes 16, 17, 18 prenant chacune 128 positions différentes, le système nécessite 384 séquences d'acquisition distinctes. Le milieu étudié peut alors être segmenté en $128^3$ voxels 19 de forme cubique. La cadence d'acquisition des signaux ultrasonores est comprise entre 100 et 100 000 tirs par seconde.

Si l'on suppose que dans le milieu étudié, les ondes de cisaillement se propage à 1 m/s et que la dimension principale de ce milieu soit 12.8 cm et que les voxels aient pour dimension 1mm$^3$. La propagation de l'onde de cisaillement dans un tel milieu et sur une longueur de 12.8 cm dure 128 ms. Pour une cadence typique de 1 000 tirs par seconde, 128 tirs ultrasonores devront être réalisés pour suivre la propagation de l'onde de cisaillement. On peut alors estimer qu'au bout de 150 ms l'acquisition se termine. Supposons que le dispositif ultrasonore se déplace au bout de 500 ms et qu'une deuxième série de 128 tirs ultrasonores soit réalisée. Si on utilise trois barrettes ultrasonores afin d'accéder aux trois composantes du déplacement, il faudra environ 3 minutes (384 fois 500 ms) pour acquérir l'ensemble des données nécessaires à la résolution du problème inverse. Ce temps de mesure peut être diminué en entrelaçant les tirs ultrasonores, 128 tirs seraient nécessaires donc une minute d'acquisition.

En cas de balayage, une des difficultés consiste à garder un bon couplage entre le transducteur et le milieu étudié pendant toute la durée du balayage. Dans le cas où la surface du milieu est plane, le balayage peut être réalisé en utilisant un couplant ultrasonore, par exemple un gel à base d'eau. Lorsque ce n'est pas possible ou lorsque la surface du milieu est « accidentée » nous proposons d'immerger le milieu viscoélastique dans de l'eau. Ce cas de figure est représenté sur la figure 6 où le sein 21 d'une patiente est immergé dans un réservoir 22, parallélépipédique comportant des fenêtres transparentes aux ultrasons, rempli d'eau.

Comme nous l'avons vu précédemment, le dispositif selon l'invention nécessite au moins une barrette échographique. Elle nécessite également un équipement électronique pour l'acquisition ultrasonore constitué d'émetteurs et de récepteurs ultrasonores, de convertisseurs numérique-analogique et analogique-numérique, de mémoires, de lignes de transmission numérique et analogique, etc. À cette électronique dédiée à la numérisation des signaux ultrasonores, on ajoute en général une unité de traitement qui peut être par exemple un ordinateur de type PC associé à une interface utilisateur. Les éléments mentionnés dans ce paragraphe ne sont pas représentés sur les différentes figures mais sont parfaitement connus de l'homme du métier.

Les techniques d'imagerie ultrasonore ultrarapide n'utilisent en général qu'un nombre limité d'émissions ultrasonores pour illuminer l'ensemble du milieu à imager. Elles présentent donc l'inconvénient d'envoyer moins d'énergie dans le milieu qu'un système échographique standard. En conséquence le rapport signal sur bruit chute et la dynamique de l'image ultrasonore diminue, ce qui entraîne une dégradation des données ultrasonores brutes et se répercutent dans la chaîne d'algorithmes pour dégrader les mesures d'élasticité en terme de sensibilité, de résolution, etc.

Pour pallier cet inconvénient, le dispositif de l'invention embarque une partie du susdit équipement électronique à proximité, c'est-à-dire typiquement à une distance inférieure à 50 centimètres, de la barrette ultrasonore avec comme conséquences :

- l'augmentation de la sensibilité du système,
- l'augmentation de l'énergie transmise,
- la simplification des connexions entre la partie capteur motorisé (barrette + équipement électronique embarqué) et l'unité de traitement des données (PC ou carte PC embarquée ou processeur DSP ou etc.),
- une plus grande immunité au bruit.

Ces modifications entraînent une diminution de la mobilité de la barrette qui ne serait pas compatible avec une utilisation standard en échographie car les barrettes échographiques doivent être légères et maniables. Il est important de noter que dans le cas qui nous concerne, la mobilité de la barrette est de toute façon limitée par la course du balayage. Le poids de la barrette a une importance moindre car la barrette n'est pas manipulée. Elle est motorisée.

Selon une possibilité offerte par l'invention, le dispositif propose de placer à proximité, soit typiquement moins de 50, de la barrette la partie analogique d'émission et de réception, c'est-à-dire les amplificateurs d'émission et de réception, en conservant une transmission de signaux analogiques de niveaux moyens entre la partie capteur et l'unité de traitement. De cette façon, le trajet des signaux analogiques d'émission forts (après amplification) et celui des signaux de réception faibles (avant amplification) sont réduits, par conséquent la sensibilité en réception est augmentée et le transfert d'énergie à l'émission améliorée.

Selon une autre possibilité offerte par l'invention, le dispositif propose de placer également à proximité, toujours typiquement moins de 50 cm (centimètre), de la barrette les convertisseurs analogique-numérique (CAN) et numérique-analogique (CNA) (pour l'émission et la réception) et de relier la partie capteur et l'unité de traitement par une liaison numérique à très haute vitesse (de type LVDS par exemple). La structure du dispositif selon l'invention ainsi réalisée entraîne les améliorations suivantes :

- le rapport signal sur bruit est augmenté en logeant toute la partie analogique au niveau de la source. Les signaux analogiques forts (émetteurs) et faibles (récepteurs) sont concentrés au niveau du capteur et ne parcourent plus la distance entre l'unité de traitement et la partie capteur.
- Le bruit reçu et le bruit émis sont diminués car la connexion entre l'unité de traitement et la partie capteur devient purement numérique.

La connexion entre le moyen de commande/traitement et la partie capteur est simplifiée en terme de nombre de fils.

Si l'on suppose qu'un opérateur utilise une barrette de 128 éléments, des convertisseurs (CNA et CAN) 8 bits à 50 MHz pour l'émission et la réception. Si émission et réception sont séparées dans le temps et que toutes les voies sont actives, le taux de transfert de données numériques atteint $128 \times 8 \times 50 = 51,2$ Gbps (Giga bits par seconde). Il suffit actuellement de 17 connexions numériques haute vitesse à 3,125 Gbps pour transmettre ces données en temps réel. Par comparaison une solution analogique nécessiterait 128 connexions bifilaires.

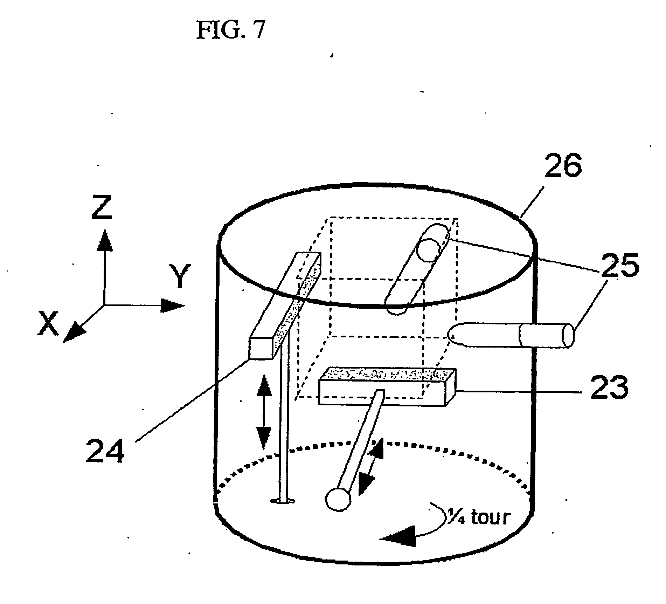

Sur la figure 7, le dispositif selon l'invention est représenté dans un nouveau montage. Dans cette variante du dispositif, deux sondes ultrasonores 23 et 24 sont utilisées, et sont immergées dans un récipient hermétique 26, rempli d'eau ou d'un autre liquide adéquat.

Le récipient ou boîtier hermétique est apte à tourner, par exemple d'un quart de tour, de sorte que la sonde 23 puisse non seulement balayer suivant la direction X mais également suivant la direction Z. La sonde ultrasonore 24 balaye uniquement suivant la direction Z. L'acquisition des signaux ultrasonores se fait donc en trois temps :

- balayage suivant les directions X et Z grâce aux deux sondes 23 et 24,
- rotation du récipient ou boîtier hermétique 26 par exemple d'un quart de tour, soit 90 degrés,
- balayage suivant la direction Z, grâce à la sonde 23.

Pendant les acquisitions, des vibreurs mécaniques 25, insérés dans des orifices présents sur le pourtour ou la circonférence du récipient hermétique 26, peuvent être utilisés pour engendrer des sollicitations basses fréquence. L'un des deux vibreurs mécaniques, ou les deux vibreurs mécaniques, représentés sur la figure 7 peut/peuvent être remplacé(s) par une sonde d'hyperthermie et/ou un transducteur ultrasonore utilisé en mode palpation à distance. Dans l'exemple choisi pour illustrer l'invention, les deux orifices présents dans ou sur le boîtier hermétique 26 sont situés à 90 degrés l'un de l'autre, c'est-à-dire que les vibreurs mécaniques linéaires seront disposés perpendiculaire l'un par rapport à l'autre, de manière à ce que même après une rotation d'un quart de tour (90°) du récipient 26, les vibreurs mécaniques s'étendent toujours suivant les mêmes directions, c'est-à-dire les mêmes droites qu'au préalable.

L'invention est décrite dans ce qui précède à titre d'exemple. Il est entendu que l'homme du métier est à même de réaliser différentes variantes du dispositif et du procédé pour la mesure de l'élasticité d'un organe humain ou animal et l'établissement consécutif d'une représentation à deux ou trois dimensions de l'élasticité, en particulier concernant la disposition ou l'agencement des différents éléments constituant ledit dispositif ou l'ordre ainsi que l'importance des étapes dudit procédé, sans pour autant sortir du cadre du brevet.

**Revendications**

**1.** Dispositif pour la mesure de l'élasticité d'un organe humain ou animal, en particulier d'un sein, présentant un signal ultrasonore après illumination ultrasonore et l'établissement consécutif d'une image à deux dimensions de l'élasticité, comprenant au moins une barrette ultrasonore (1) comprenant une pluralité de transducteurs (12), un moyen d'excitation générant une excitation basse fréquence comprise entre 5 Hz et 1000 Hz, un moyen de balayage pour effectuer un balayage de la barrette (1), un moyen d'acquisition de signaux ultrasonores, un moyen de commande et de traitement de données, ledit moyen de commande et de traitement des données déterminant la vitesse des tissus biologiques dans le plan de l'image en utilisant les signaux ultrasonores en des points situés de part et d'autre du plan de l'image.

**2.** Dispositif selon la revendication 1, **caractérisé en ce qu'**il comprend des moyens de balayage pour focaliser le faisceau ultrasonore de ladite barrette ultrasonore (1) de part et d'autre du plan de l'image selon un des procédés suivants :

- un déplacement mécanique de ladite barrette ultrasonore (1) selon une direction perpendiculaire au plan de l'image ;
- un déplacement mécanique de ladite barrette ultrasonore (1) et un déplacement mécanique d'une barrette ultrasonore additionnelle (6), ledit dispositif (1) comportant ladite barrette ultrasonore additionnelle (6) ;
- une modification électronique de la focalisation de ladite barrette ultrasonore (1).

**3.** Dispositif selon l'une au moins des revendications précédentes **caractérisé en ce qu'**une pluralité d'images sont combinées de façon à obtenir une représentation à trois dimensions de l'élasticité.

**4.** Dispositif selon la revendication 1, **caractérisé en ce que** le moyen d'excitation consiste en une vibration mécanique, qui pourra être transversale, longitudinale ou plus généralement un mélange des deux.

**5.** Dispositif selon la revendication 1, **caractérisé en ce que** le moyen d'excitation consiste en une palpation à distance en utilisant la pression de radiation, soit avec le (s) transducteur (s) (12) utilisé (s) pour l'acquisition des signaux ultrasonores soit un ou plusieurs transducteurs disposés autour du milieu viscoélastique.

**6.** Dispositif selon la revendication 1, **caractérisé en ce que** le moyen d'excitation consiste en des mouvements internes du corps humain ou animal, tels que par exemple les battements cardiaques.

**7.** Dispositif selon la revendication 1, **caractérisé en ce que** le moyen d'excitation consiste en un ou plusieurs transducteurs d'hyperthermie, soit avec le (s) transducteur (s) utilisé (s) pour l'acquisition des signaux ultrasonores soit un ou plusieurs transducteurs disposés autour du milieu viscoélastique.

8.  Dispositif selon la revendication 1, **caractérisé en ce que** la barrette ultrasonore est un transducteur étoilé (12) permettant de focaliser en une pluralité de différents points d'élévation.

9.  Dispositif selon la revendication 1, **caractérisé en ce que** l'espace existant entre la barrette ultrasonore (1) et le susdit milieu viscoélastique est constitué au moins en partie par de l'eau ou tout autre élément apte à assurer le libre passage des ondes ultrasonores.

10. Dispositif selon la revendication 4, **caractérisé en ce que** la vibration mécanique est obtenue ou réalisée grâce à une ou plusieurs plaques vibrantes (20), piston (s) et/ou barre (s).

11. Dispositif selon la revendication 1, **caractérisé en ce que** le moyen d'acquisition comprend des émetteurs et des récepteurs ultrasonores, des convertisseurs numérique-analogique (CNA) et analogique-numérique (CAN), des mémoires, des lignes de transmission numérique et analogique.

12. Dispositif selon la revendication 11, **caractérisé en ce que** les émetteurs et les récepteurs ultrasonores sont disposés à proximité de la susdite barrette ultrasonore, soit typiquement une distance inférieure à 50 centimètres.

13. Dispositif selon la revendication 11, **caractérisé en ce que** les convertisseurs numérique-analogique (CNA) et analogique-numérique (CAN) sont situés à proximité de la susdite barrette ultrasonore, soit une distance inférieure à 50 centimètres.

14. Dispositif selon la revendication 11, **caractérisé en ce que** l'ensemble constitué des transducteurs ultrasonores et de leur électronique embarquée est relié au moyen de commande et de traitement par une liaison numérique à très haute vitesse, par exemple de type LVDS.

15. Dispositif selon la revendication 1, **caractérisé en ce qu'**il comprend trois barrettes (16, 17,18) aptes respectivement à mesurer les vitesses tissulaires suivant les directions y, x et z.

16. Dispositif selon la revendication 2, **caractérisé en ce que** les deux barrettes (1 et 6) sont immergées dans un récipient hermétique (26) rempli d'un liquide, par exemple de l'eau.

17. Dispositif selon la revendication 16, **caractérisé en ce que** le récipient hermétique (26) est relié à un moyen de rotation apte à faire tourner ledit récipient (26).

18. Dispositif selon la revendication 16, **caractérisé en ce que** le récipient hermétique (26) comprend une pluralité d'orifices dans lesquels sont introduits respectivement un vibreur mécanique (25) et/ou un transducteur ultrasonore.

19. Dispositif selon la revendication 18, **caractérisé en ce que** les orifices sur ou dans le boîtier hermétique (26) sont situés à 90° (degrés) les uns des autres ou l'un de l'autre.

20. Procédé pour la mesure de l'élasticité d'un organe humain ou animal, en particulier d'un sein, en utilisant un dispositif selon l'une des revendications 1 à 19, ledit procédé comportant les étapes suivantes :

    - génération d'une sollicitation, ou signal, basse fréquence comprise entre 5 Hz et 1000 Hz et acquisition de signaux ultrasonores,
    - déplacements de la barrette grâce au moyen de balayage, suivant deux directions perpendiculaires,
    - calcul des images ultrasonores,
    - calcul des vitesses tissulaires,
    - inversion des données consistant à récupérer les paramètres qui décrivent ledit milieu viscoélastique.

21. Procédé selon la revendication 20, **caractérisé en ce qu'**il comprend en outre une étape de calcul des vitesses de déformation tissulaire.

22. Procédé selon la revendication 20, **caractérisé en ce qu'**au cours de l'étape de-calcul des vitesses tissulaires, les dérivées seconde de la composante longitudinale de ladite vitesse suivant les trois directions orthogonales de l'espace sont mesurées.

23. Procédé selon la revendication 20, **caractérisé en ce qu'**au cours de l'étape de calcul des vitesses tissulaires, les dérivées spatiales des trois composantes, suivant les trois directions de l'espace, de ladite vitesse sont mesurées.

24. Procédé selon la revendication 20, **caractérisé en ce que** l'acquisition de signaux ultrasonores s'effectue en émettant, avec les transducteurs ultrasonores (12) une impulsion qui est réfléchie par les particules contenues dans le milieu viscoélastique.

25. Procédé selon les revendications 20 et 24, **caractérisé en ce que** l'acquisition de signaux ultrasonores est réalisée à une cadence 1/T, typiquement comprise entre 100 Hz et 100 000 Hz, T étant la période entre deux émissions ultrasonores.

**Claims**

1.  Device for measuring the elasticity of a human or animal organ, in particular of a breast, presenting an ultrasonic signal after ultrasonic illumination and the consecutive establishing of an image in two dimensions of the elasticity, comprising at least one ultrasonic bar (1) comprising a plurality of transducers (12), an excitation means generating a low-frequency excitation comprised between 5 Hz and 1000 Hz, a scanning means for carrying out a scanning of the bar (1), a means for acquisition of ultrasonic signals, a means for the control and processing of data, the said means for the control and processing of data determining the speed of the biological tissues in the plane of the image by using the ultrasonic signals at points situated on both sides of the plane of the image.

2.  Device according to claim 1, **characterized in that** it comprises scanning means for focussing the ultrasonic beam of the said ultrasonic bar (1) on both sides of the plane of the image according to one of the following processes:

    - a mechanical displacement of the said ultrasonic bar (1) according to a direction perpendicular to the plane of the image;
    - a mechanical displacement of the said ultrasonic bar (1) and a mechanical displacement of an additional ultrasonic bar (6), the said device (1) comprising the said additional ultrasonic bar (6);
    - an electronic modification of the focussing of the said ultrasonic bar (1).

3.  Device according to at least one of the preceding claims, **characterized in that** a plurality of images are combined so as to obtain a representation in three dimensions of the elasticity.

4.  Device according to claim 1, **characterized in that** the excitation means consists of a mechanical vibration which will be able to be transverse, longitudinal or more generally a mixture of the two.

5.  Device according to claim 1, **characterized in that** the excitation means consists in a remote palpation by using pressure of radiation, either with the transducer(s) (12) used for the acquisition of the ultrasonic signals or one or several transducers arranged around the viscoelastic environment.

6.  Device according to claim 1, **characterized in that** the excitation means consists in internal movements of the human or animal body, such as for example the heartbeats.

7.  Device according to claim 1, **characterized in that** the excitation means consists in one or several hyperthermal transducers, either with the transducer(s) used for the acquisition of the ultrasonic signals or one or several transducers arranged around the viscoelastic environment.

8.  Device according to claim 1, **characterized in that** the ultrasonic bar is a starred transducer (12) permitting focussing in a plurality of different elevation points.

9.  Device according to claim 1, **characterized in that** the space existing between the ultrasonic bar (1) and the above-mentioned viscoelastic environment is constituted at least in part by water or any other element capable of ensuring free passage of the ultrasonic waves.

10. Device according to claim 4, **characterized in that** the mechanical vibration is obtained or realized owing to one or several vibrating plates (20), piston(s) and/or bar(s).

11. Device according to claim 1, **characterized in that** the acquisition means comprises ultrasonic transmitters and receivers, digital-to-analog converters (DAC) and analog-to-digital converters (ADC), memories, digital and analog transmission lines.

12. Device according to claim 11, **characterized in that** the ultrasonic transmitters and receivers are arranged close to the above-mentioned ultrasonic bar, namely typically at a distance less than 50 centimetres.

13. Device according to claim 11, **characterized in that** the digital-to-analog converters (DAC) and analog-to-digital converters (ADC) are situated close to the above-mentioned ultrasonic bar, namely at a distance less than 50 centimetres.

14. Device according to claim 11, **characterized in that** the unit constituted by the ultrasonic transducers and their on-board electronics is connected to the control and processing means by a very high speed digital connection, for example of the LVDS type.

15. Device according to claim 1, **characterized in that** it comprises three bars (16, 17, 18) suited respectively to measuring the tissue speeds according to the directions y, x and z.

16. Device according to claim 2, **characterized in that** the two bars (1 and 6) are immersed in a hermetic container (26) filled with a liquid, for example with water.

17. Device according to claim 16, **characterized in that** the hermetic container (26) is connected to a rotation means suited to rotate the said container (26).

18. Device according to claim 16, **characterized in that** the hermetic container (26) comprises a plurality of orifices into which are introduced respectively a mechanical vibrator (25) and/or an ultrasonic transducer.

19. Device according to claim 18, **characterized in that** the orifices on or in the hermetic container (26) are situated at 90° (degrees) from each other or one from the other.

20. Process for measuring the elasticity of a human or animal organ, in particular of a breast, using a device according to one of claims 1 to 19, the said process comprising the following steps:

- generating a low-frequency stress, or signal, comprised between 5Hz and 1000 Hz and acquiring ultrasonic signals,
- displacing the bar by the scanning means, according to two perpendicular directions,
- calculating ultrasonic images,
- calculating tissue speeds,
- inverting the data consisting in recovering the parameters which describe the said viscoelastic environment.

21. Process according to claim 20, **characterized in that** it further comprises a calculating step of the tissue deformation speeds.

22. Process according to claim 20, **characterized in that** in the course of the step of calculation of the tissue speeds, the second derivatives of the longitudinal component of the said speed are measured according to the three orthogonal directions of the space.

23. Process according to claim 20, **characterized in that** in the course of the step of calculation of the tissue speeds, the spatial derivatives of the three components, according to the three directions of the space, of the said speed are measured.

24. Process according to claim 20, **characterized in that** the acquisition of ultrasonic signals is carried out by emitting, with the ultrasonic transducers (12), an impulse which is reflected by the particles contained in the viscoelastic environment.

25. Process according to claims 20 and 24, **characterized in that** the acquisition of ultrasonic signals is carried out at a cadence 1/T, typically comprised between 100 Hz and 100 000 Hz, T being the period between two ultrasonic emissions.

**Patentansprüche**

1. Vorrichtung zur Messung der Elastizität eines menschlichen oder tierischen Organs, insbesondere einer Brust, welches nach Ultraschallbeleuchtung ein Ultraschallsignal aufweist, und zur anschließenden Erstellung eines zweidimensionalen Bildes der Elastizität, welche umfasst: mindestens einen Ultraschallstab (1), der mehrere Messwandler (12) umfasst, ein Erregungsmittel, das eine niederfrequente Erregung im Bereich von 5 Hz bis 1000 Hz erzeugt, ein Abtastmittel zur Durchführung einer Abtastung des Stabes (1), ein Mittel zur Erfassung von Ultraschallsignalen, ein Mittel zur Steuerung und Verarbeitung von Daten, wobei das Mittel zur Steuerung und Verarbeitung der Daten die Geschwindigkeit der biologischen Gewebe in der Bildebene unter Verwendung der Ultraschallsignale in Punkten bestimmt, die sich beiderseits der Bildebene befinden.

2. Vorrichtung nach Anspruch 1, **dadurch gekennzeichnet, dass** sie Abtastmittel umfasst, um das Ultraschallbündel des Ultraschallstabes (1) beiderseits der Bildebene nach einem der folgenden Verfahren zu fokussieren:

   - mechanische Verschiebung des Ultraschallstabes (1) in einer Richtung, die zur Bildebene senkrecht ist;
   - mechanische Verschiebung des Ultraschallstabes (1) und mechanische Verschiebung eines zusätzlichen Ultraschallstabes (6), wobei die Vorrichtung (1) den zusätzlichen Ultraschallstab (6) aufweist;
   - elektronische Veränderung der Fokussierung des Ultraschallstabes (1).

3. Vorrichtung nach wenigstens einem der vorhergehenden Ansprüche, **dadurch gekennzeichnet, dass** mehrere Bilder kombiniert werden, derart, dass eine dreidimensionale Darstellung der Elastizität erhalten wird.

4. Vorrichtung nach Anspruch 1, **dadurch gekennzeichnet, dass** das Erregungsmittel aus einer mechanischen Schwingung besteht, welche eine Querschwingung, eine Längsschwingung oder, allgemeiner, eine Kombination von beidem sein kann.

5. Vorrichtung nach Anspruch 1, **dadurch gekennzeichnet, dass** das Erregungsmittel aus einer Fernabtastung unter Verwendung des Strahlungsdruckes entweder mit dem (den) Messwandler(n) (12), der (die) für die Erfassung der Ultraschallsignale verwendet wird (werden), oder mit einem oder mehreren Messwandlern, die um das viskoelastische Medium herum angeordnet sind, besteht.

6. Vorrichtung nach Anspruch 1, **dadurch gekennzeichnet, dass** das Erregungsmittel aus inneren Bewegungen des menschlichen oder tierischen Körpers besteht, wie zum Beispiel Herzschlägen.

7. Vorrichtung nach Anspruch 1, **dadurch gekennzeichnet, dass** das Erregungsmittel aus einem oder mehreren Hyperthermie-Messwandlern besteht, entweder mit dem (den) Messwandler(n), der (die) für die Erfassung der Ultraschallsignale verwendet wird (werden), oder mit einem oder mehreren Messwandlern, die um das viskoelastische Medium herum angeordnet sind.

8. Vorrichtung nach Anspruch 1, **dadurch gekennzeichnet, dass** der Ultraschallstab ein sternförmig konfigurierter Messwandler (12) ist, der eine Fokussierung auf mehrere verschiedene Höhenpunkte ermöglicht.

9. Vorrichtung nach Anspruch 1, **dadurch gekennzeichnet, dass** der Raum, der zwischen dem Ultraschallstab (1) und dem viskoelastischen Medium vorhanden ist, mindestens teilweise aus Wasser oder einem beliebigen anderen Element besteht, das geeignet ist, den freien Durchgang der Ultraschallwellen zu gewährleisten.

10. Vorrichtung nach Anspruch 4, **dadurch gekennzeichnet, dass** die mechanische Schwingung durch eine oder mehrere Rüttelplatten (20), Kolben und/oder Stangen erzielt oder erzeugt wird.

11. Vorrichtung nach Anspruch 1, **dadurch gekennzeichnet, dass** das Mittel zur Erfassung Ultraschallsender und -empfänger, Digital-Analog-Umsetzer (DAU) und Analog-Digital-Umsetzer (ADU), Speicher sowie digitale und analoge Übertragungsleitungen umfasst.

12. Vorrichtung nach Anspruch 11, **dadurch gekennzeichnet, dass** die Ultraschallsender und -empfänger in der Nähe des Ultraschallstabes angeordnet sind, und zwar typischerweise in einem Abstand von weniger als 50 Zentimetern.

13. Vorrichtung nach Anspruch 11, **dadurch gekennzeichnet, dass** die Digital-Analog-Umsetzer (DAU) und Analog-Digital-Umsetzer (ADU) in der Nähe des Ultraschallstabes angeordnet sind, und zwar in einem Abstand von weniger als 50 Zentimetern.

14. Vorrichtung nach Anspruch 11, **dadurch gekennzeichnet, dass** die aus den Ultraschallwandlern und deren integrierter Elektronik bestehende Baueinheit mit dem Mittel zur Steuerung und Verarbeitung durch eine digitale Verbindung mit sehr hoher Geschwindigkeit, zum Beispiel vom Typ LVDS, verbunden ist.

15. Vorrichtung nach Anspruch 1, **dadurch gekennzeichnet, dass** sie drei Stäbe (16, 17, 18) umfasst, die geeignet sind, die Gewebegeschwindigkeiten in den Richtungen y, x bzw. z zu messen.

16. Vorrichtung nach Anspruch 2, **dadurch gekennzeichnet, dass** die zwei Stäbe (1 und 6) in einen hermetischen Behälter (26) eingetaucht sind, der mit einer Flüssigkeit, zum Beispiel Wasser, gefüllt ist.

17. Vorrichtung nach Anspruch 16, **dadurch gekennzeichnet, dass** der hermetische Behälter (26) mit einem Rotationsmittel verbunden ist, das geeignet ist, den Behälter (26) zu drehen.

18. Vorrichtung nach Anspruch 16, **dadurch gekennzeichnet, dass** der hermetische Behälter (26) mehrere Öffnungen umfasst, in welche jeweils ein mechanischer Vibrator (25) und/oder ein Ultraschallwandler eingesetzt sind.

19. Vorrichtung nach Anspruch 18, **dadurch gekennzeichnet, dass** die Öffnungen an oder in dem hermetischen Gehäuse (26) in einem Abstand von 90° (Grad) voneinander angeordnet sind.

20. Verfahren zur Messung der Elastizität eines menschlichen oder tierischen Organs, insbesondere einer Brust, unter Verwendung einer Vorrichtung nach einem der Ansprüche 1 bis 19, wobei das Verfahren die folgenden Schritte aufweist:

   - Erzeugung einer Beanspruchung, oder eines Signals, mit niedriger Frequenz im Bereich von 5 Hz bis 1000 Hz und Erfassung von Ultraschallsignalen,
   - Verschiebung des Stabes mit Hilfe des Abtastmittels in zwei zueinander senkrechten Richtungen,
   - Berechnung der Ultraschallbilder,
   - Berechnung der Gewebegeschwindigkeiten,
   - Inversion der Daten, darin bestehend, die Parameter zurückzugewinnen, welche das viskoelastische Medium beschreiben.

21. Verfahren nach Anspruch 20, **dadurch gekennzeichnet, dass** es außerdem einen Schritt der Berechnung der Geschwindigkeiten der Gewebeverformung umfasst.

22. Verfahren nach Anspruch 20, **dadurch gekennzeichnet, dass** während des Schrittes der Berechnung der Gewebegeschwindigkeiten die Ableitungen der longitudinalen Komponente der Geschwindigkeit nach der Zeit in den drei orthogonalen Richtungen des Raumes gemessen werden.

23. Verfahren nach Anspruch 20, **dadurch gekennzeichnet, dass** während des Schrittes der Berechnung der Gewebegeschwindigkeiten die räumlichen Ableitungen der drei Komponenten der Geschwindigkeit in den drei Richtungen des Raumes gemessen werden.

24. Verfahren nach Anspruch 20, **dadurch gekennzeichnet, dass** die Erfassung von Ultraschallsignalen durchgeführt wird, indem mit den Ultraschallwandlern (12) ein Impuls ausgesendet wird, welcher von den in dem viskoelastischen Medium enthaltenen Teilchen reflektiert wird.

25. Verfahren nach den Ansprüchen 20 und 24, **dadurch gekennzeichnet, dass** die Erfassung von Ultraschallsignalen in einem Zeittakt 1/T durchgeführt wird, der typischerweise im Bereich von 100 Hz bis 100 000 Hz liegt, wobei T das Zeitintervall zwischen zwei Ultraschallemissionen ist.

## FIGURE 1

## FIG. 2

## FIG. 3

FIG. 4

FIG. 5

FIG. 6

## FIG. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2733142
- US 6176827 B
- US 5099848 A
- US 2002010398 A
- US 6277074 B
- US 5474070 A
- WO 0070362 A
- WO 0055616 A
- FR 9903157

### Littérature non-brevet citée dans la description

- **SANDRIN, L. ; TANTER, M. ; CATHELINE, S. ; FINK, M.** Shear Modulus Imaging with 2D Transient Elastography. *Ultrason. Ferroelectr. Freq. Control.,* 2002, vol. 49 (4), 426-435
- **LU, J.** 2D and 3D High Frame Rate Imaging with Limited Diffraction Beams. *IEEE Trans. Ultrason. Ferroelectr. Freq. Contr.,* 1997, vol. 44 (4
- **KASAI C. ; NAMEKAWA K. ; KOYANO A. ; OMOTO R.** Real-time two-dimensional blood flow imaging using an autocorrelation technique. *IEEE Trans. Sonics Ultrason.,* 1985, vol. 35, 458-464
- **KONOFAGOU, E. E. ; OPHIR, J.** A new elastographic method for estimation and imaging of lateral displacements, lateral strains, corrected axial strains and Poisson's ratios in tissues. *Ultrasound in Med. & Biol.,* 1998, vol. 24 (8), 1183-1199
- **TANTER, M. ; BERCOFF, J. ; SANDRIN, L. ; FINK, M.** Ultrafast compound imaging for 2D motion vector estimation : application to transient elastography. *Ultrason. Ferroelectr. Freq. Control.*